(19) Europäisches Patentamt
European Patent Office
Office européen des brevets

(11) EP 3 157 906 B1

(12) # EUROPEAN PATENT SPECIFICATION

(45) Date of publication and mention of the grant of the patent:
**27.02.2019 Bulletin 2019/09**

(21) Application number: **15731028.5**

(22) Date of filing: **18.06.2015**

(51) Int Cl.:
***C07D 231/54*** (2006.01) ***C07D 271/04*** (2006.01)
***C07D 495/04*** (2006.01)

(86) International application number:
**PCT/EP2015/063755**

(87) International publication number:
**WO 2015/193455 (23.12.2015 Gazette 2015/51)**

(54) **IMINOSYDNONE DERIVATIVES FOR CONJUGATION AND RELEASE OF COMPOUNDS OF INTEREST**

IMINOSYDNONDERIVATE ZUR KONJUGATION UND FREISETZUNG VON VERBINDUNGEN VON INTERESSE

DÉRIVÉS IMINOSYDNONES POUR LA CONJUGAISON ET LA LIBÉRATION DE COMPOSÉS D'INTÉRÊT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2014 EP 14305933**

(43) Date of publication of application:
**26.04.2017 Bulletin 2017/17**

(73) Proprietors:
- **Université de Strasbourg**
  **67000 Strasbourg (FR)**
- **Centre National de la Recherche Scientifique**
  **75016 Paris (FR)**
- **Commissariat à l'Energie Atomique et aux Energies Alternatives**
  **75015 Paris (FR)**

(72) Inventors:
- **TARAN, Frédéric**
  **F-91190 Gif Sur Yvette (FR)**
- **WAGNER, Alain**
  **F-67000 Strasbourg (FR)**
- **KOLODYCH, Sergii**
  **F-67000 Strasbourg (FR)**
- **KONIEV, Oleksandr**
  **F-67000 Strasbourg (FR)**

(74) Representative: **Cabinet Becker et Associés**
**25, rue Louis le Grand**
**75002 Paris (FR)**

(56) References cited:
**US-A1- 2014 051 660 US-B1- 6 545 033**

- **DAENIKER; DRUEY: "Heilmittelchemische Studien In Der Heterocyclischen Reihe. 36. Mitteilung. Über Sydnonimine. II. Herstellung und Eigenschaften von Sydnoniminen mit Acyl-, Carbamoyl- und Thiocarbamoyl-Substituenten am exocyclischen Stickstoffatom", HELV. CHIM. ACTA, vol. 45, no. 7, 1 January 1962 (1962-01-01), pages 2441-2462, XP055152550, ISSN: 0018-019X, DOI: 10.1002/hlca.19620450714**
- **DATABASE CA [Online] CHEMICAL ABSTRACTS SERVICE, COLUMBUS, OHIO, US; YASHUNSKII, V. G. ET AL: "Sydnones and sydnonimines. XXVI. Reactivity of 3-aryl-sydnonimines", XP002732477, retrieved from STN Database accession no. 1966:59381 & COLLECTION OF CZECHOSLOVAK CHEMICAL COMMUNICATIONS , 30(12), 4257-71., 1965,**
- **DATABASE CA [Online] CHEMICAL ABSTRACTS SERVICE, COLUMBUS, OHIO, US; KAMITANI, TAKASHI ET AL.: "Sydnonimines", XP002732478, retrieved from STN Database accession no. 1971:13164 & JP S45 26086 B (FUJISAWA PHARMACEUTICAL CO., LTD.) 28 August 1970 (1970-08-28)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 157 906 B1

- DATABASE CA [Online] CHEMICAL ABSTRACTS SERVICE, COLUMBUS, OHIO, US; KAMITANI, TAKASHI ET AL: "Sydnonimines", XP002732479, retrieved from STN Database accession no. 1971:13161 & JP S45 26088 B (FUJISAWA PHARMACEUTICAL CO., LTD.) 28 August 1970 (1970-08-28)
- DATABASE CA [Online] CHEMICAL ABSTRACTS SERVICE, COLUMBUS, OHIO, US; KAMITANI, TAKASHI ET AL: "N-Substituted-syndnonimines", XP002732480, retrieved from STN Database accession no. 1971:13160 & JP S45 26090 B (FUJISAWA PHARMACEUTICAL CO., LTD.) 28 August 1970 (1970-08-28)
- BROWNE; HARRITY: "Recent developments in the chemistry of sydnones", TETRAHEDRON, vol. 66, no. 3, 16 January 2010 (2010-01-16), pages 553-568, XP027534268, ISSN: 0040-4020 [retrieved on 2009-10-29]
- WALLACE; CHIN: "Strain-promoted sydnone bicyclo-[6.1.0]-nonyne cycloaddition", CHEMICAL SCIENCE, vol. 5, no. 5, 5 February 2014 (2014-02-05), page 1742, XP055142993, ISSN: 2041-6520, DOI: 10.1039/c3sc53332h
- KOLODYCH ET AL.: "Discovery of Chemoselective and Biocompatible Reactions Using a High-Throughput Immunoassay Screening", ANGEW. CHEM. INT. ED., vol. 52, no. 46, 7 October 2013 (2013-10-07), pages 12056-12060, XP055142984, ISSN: 1433-7851, DOI: 10.1002/anie.201305645 cited in the application
- VINATIER ET AL.: "Esterase-activated chromane-sydnonimine prodrug hybrids", NITRIC OXIDE, vol. 15, no. 4, 1 December 2006 (2006-12-01), pages 363-369, XP024919018, ISSN: 1089-8603, DOI: 10.1016/J.NIOX.2006.03.006 [retrieved on 2006-12-01]
- NAGAKURA ET AL: "Syntheses and antiinflammatory actions of 4,5,6,7-tetrahydroindazole-5- carboxylic acids", J. MED. CHEM., vol. 22, no. 1, 1 January 1979 (1979-01-01), pages 48-52, XP002337914, ISSN: 0022-2623, DOI: 10.1021/JM00187A012
- PLOUGASTEL ET AL.: "4-Halogeno-sydnones for fast strain promoted cycloaddition with bicyclo-[6.1.0]-nonyne", CHEM. COMM., vol. 50, no. 66, 30 June 2014 (2014-06-30) , pages 9376-9378, XP055143131, ISSN: 1359-7345, DOI: 10.1039/C4CC03816A
- Angew. Chem. Int. Ed. 2010, 49, 9422-9425.
- Chem. Commun. 2013, 49, 11007-11022.


Remarks:

The file contains technical information submitted after the application was filed and not included in this specification

## Description

### Introduction

The present invention relates to the use of iminosydnone compounds in processes for the preparation of conjugates of two compounds of interest. The invention further relates to the use of said iminosydnone compounds in a process for releasing a third compound of interest. The invention finally relates to said new iminosydnone compounds.

### Background of the invention

The present invention relates to the domain of click chemistry (bioorthogonal reaction) and of bioconjugation of compounds of interest. Five main approaches are currently available for metal-free coupling two different compounds of interest (● and ■). These approaches are presented on the scheme below. Among these approaches only Tetrazine/Cyclooctene reaction (approach 2) has a modification that allows simultaneous coupling of two entities and release of the third compound (Angew. Chem. Int. Ed. 2013, 52, 14112-14116).

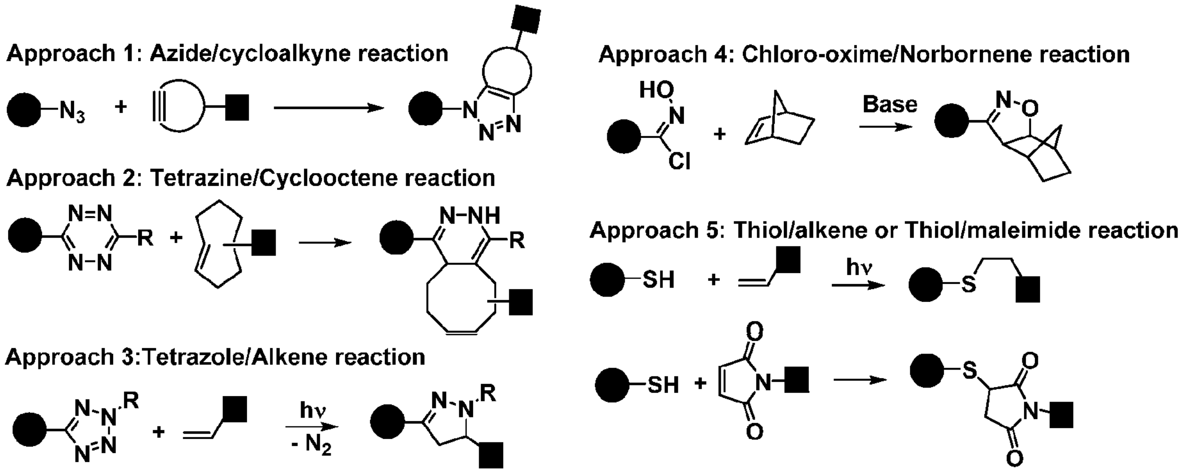


The inventors of the present application recently discovered that reaction of a sydnone and an alkyne could be efficiently used to perform efficient bioorthogonal reactions (Kolodych et al. Angew. Chem. Int. Ed. 2013, 52, 12056-12060). Furthermore, strain-promoted reactions between N-arylated sydnones and bicyclo[6.1.0]nonyne for bioorthogonal protein labelling are known in the art (Wallace, Chin Chem. Sci. 2014, 5, 1742-1744).

Bioconjugation reactions and cleavage reactions, for instance bioconjugation and cleavage reactions involving proteins, are often performed in highly dilute conditions, for instance because of the low available amount of the starting biomaterial, or because the solubility of said starting biomaterial is low in the coupling and or cleavage conditions. Furthermore, a high level of bioorthogonality of these reactions is mandatory for applications in the fields of chemical biology and drug release which deal with *in vivo* experiments.

Consequently, it is important in the domain of bioorthogonal reactions to use efficient coupling and cleavable reagents allowing high yields and fast coupling and cleavage kinetics even in the presence of the plethora of chemical functionalities present in biological media.

Such reactions are potentially of high interest in several areas; one of them is antibody-drug-conjugate (ADC) cleavage wherein an antibody, such as an anti-tumor antibody, and a drug, such as an anticancer drug, can be selectively released with a specific probe preferably administered in a second step.

The Applicant of the present invention evidenced that the use of iminosydnone derivatives affords a unique process allowing both the conjugation and the release of compounds of interest with high efficiency, kinetics and bioorthogonality. These iminosydnone compounds can be advantageously used for the conjugation and the release of compounds of interest.

### Summary of the invention

The invention is defined by the appendant claims. The description that follows is subject to this limitation. All aspects and embodiments which are not covered by the claims are merely aspects of the present description and do not form part of the invention.

A first object of the description is a process for the preparation of a functionalized compound of interest C1 of formula (II) :

(II) 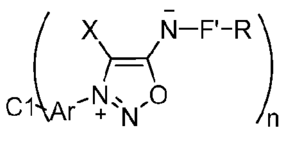 ,

comprising the step of contacting a compound of interest C1 bearing a reactive group with an iminosydnone of formula (I):

(I) 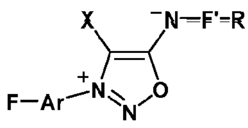 ,

wherein F is a functional group selected among specific groups which allow bonding to the reactive group of the compound of interest C1.

Another object of the description is a functionalized compound of interest C1 of formula (II):

(II) 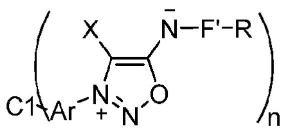 ,

An object of the invention is a conjugate of formula (IV):

(IV) 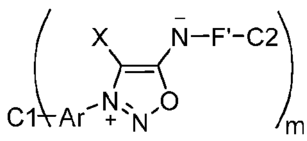 ,

wherein C1 and C2 are compounds of interest, and C1, Ar, X, F', C2 and m are as defined in claim 1.

Another object of the description is a process for the preparation of a conjugate of formula (IV):

(IV) 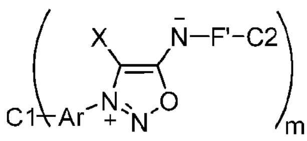 ,

comprising the step of contacting a compound of formula (II) with a compound of interest C2 bearing a reactive group which is able to react with R.

Another object of the invention is a process for the preparation of a conjugate of formula (V):

(V) 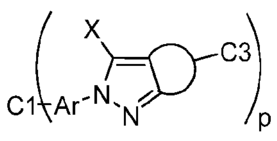

as defined in claim 1.

Another object of the description is a conjugate of formula (V):

(V) 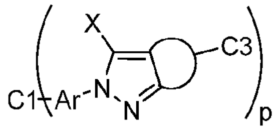,

A further object of the description is an iminosydnone of formula (I'):

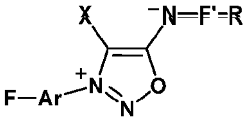 (I')

## Brief description of the figures

Figure 1: HPLC monitoring of the reaction between iminosydnone Im 5 and the cyclic alkyne TMTH.
Figure 2: HPLC monitoring (b) and linear regression curve (c) for the reaction of Im2 with TMTH (a).
Figure 3: Linear regression curves for the reactions of iminosydnones Im6 and Im7 with TMTH.

## Detailed description of the invention

A first object of the present description is a process for the preparation of a functionalized compound of interest C1 of formula (II)

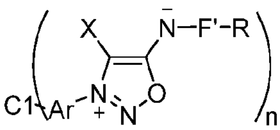 (II),

wherein n is an integer and preferably ranges from 1 to 100, comprising the step of contacting a compound of interest C1 selected from an antibody, a protein, a drug, a fluorophore, a group of atoms comprising at least one radioactive atom, a DNA fragment, a nanoparticle and a polymer, with an iminosydnone of formula (I):

(I) 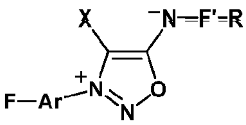,

wherein

C1 bears a reactive group which is able to react with F,
X is selected from the group consisting of a hydrogen atom, a halogen atom, an aryl diazo group, an alkyl group, an aryl group, an alkenyl group, an alkynyl group, an alkoxy group, a thioether group and an amino group,
F is a functional group selected from the group consisting of:

- a carboxylic acid COOH group,
- a thiol SH group,
- a maleimide

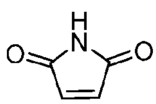

group,
- an activated ester,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR'')_2$ group, wherein R'' is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR''' group, wherein R''' is an alkyl group, and
- an alkyl group substituted by at least one of said groups,

F' is a carbonyl group (C=O), a sulfonyl group ($SO_2$) or a phosphoryl group (P=O),
R is selected from
an optionally substituted aryl group, an optionally substituted alkyl, alkenyl or alkynyl group, an optionally substituted alkoxy or aralkyloxy group, an optionally substituted thioether group, an optionally substituted amino group, wherein the alkyl, alkenyl and/or alkynyl groups may be interrupted by at least one heteroatom selected from nitrogen, oxygen and sulphur atoms and wherein said substituents are one or more groups selected from:

- a carboxylic acid COOH group,
- a thiol SH group,
- a maleimide

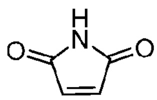

group,
- an activated ester,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR'')_2$ group, wherein R'' is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR''' group, wherein R''' is an alkyl group, or

a linker bearing at least one of the above substituents, and
Ar is an optionally substituted aromatic group.

In an embodiment, n is from 1 to 50, preferably from 1 to 30. In a specific embodiment, n is 1.
In the above definitions of F and R, the counterion can be any ion appropriate for compensating the charge of the diazonium group, and may be easily chosen by anyone of ordinary skill in the art. For instance, the couterion may be selected from the group consisting of halogenates, $BF_4$, $NO_3^-$, $HSO_4^-$, $PF_6^-$ $CH_3COO^-$, $N(SO_2CF_3)_2^-$, $CF_3SO_3^-$,

$CH_3SO_3^-$, $CF_3COO^-$, $(CH_3O)(H)PO_2^-$ and $N(CN)_2^-$.

In the context of this description, a halogen atom is a chlorine, iodine, bromine or fluorine atom. Preferably, a halogen atom is a bromine or a chlorine atom, in particular a bromine atom.

An alkyl group is a linear saturated hydrocarbon group comprising from 1 to 20 carbon atoms, a branched saturated hydrocarbon group comprising from 3 to 20 carbon atoms or a cyclic saturated hydrocarbon group comprising from 4 to 20 carbon atoms. Preferably, the alkyl group according to the invention comprises from 1 to 10, in particular from 1 to 6, carbon atoms. Examples of alkyl groups comprise methyl, ethyl, propyl, isopropyl, butyl, tertbutyl, isobutyl, n-pentyl, n-hexyl and cyclohexyl groups.

An alkenyl group (or an alkene) is a linear hydrocarbon group comprising from 2 to 20 carbon atoms, a branched hydrocarbon group comprising from 4 to 20 carbon atoms, or a cyclic hydrocarbon group comprising from 4 to 20 carbon atoms and comprising at least one C=C double bond. Preferably, the alkenyl group according to the invention comprises from 2 to 10, in particular from 2 to 6, carbon atoms. Examples of alkenyl groups comprise ethylenyl, propylenyl and cyclohexenyl groups.

An alkynyl group (or an alkyne) is a linear hydrocarbon group comprising from 2 to 20 carbon atoms, a branched hydrocarbon group comprising from 4 to 20 carbon atoms or a cyclic hydrocarbon group comprising from 4 to 20 carbon atoms and comprising at least one C=C triple bond. Preferably, the alkynyl group according to the invention comprises from 2 to 10, in particular from 2 to 9, carbon atoms. Examples of alkynyl groups comprise ethynyl, propynyl, octynyl, cyclooctynyl and cyclononynyl groups.

In specific embodiments of the present description, the alkyl, alkenyl and/or alkynyl groups may be interrupted by at least one heteroatom, preferably independently selected from nitrogen, oxygen and sulphur atoms.

An alkoxy group is an alkyl group, bonded to the rest of the molecule through an oxygen atom. An aralkyloxy group is an aralkyl group, such as a benzyl group, bonded to the rest of the molecule through an oxygen atom.

A thioether group is an alkyl group, bonded to the rest of the molecule through a sulfur atom. An aryl diazo group is a $N_2$ group bonded to an aromatic group. A carboxyl group is a COOH group. A nitro group is a $NO_2$ group.

An amino group is a $NR_1R_2$ group, wherein $R_1$ and $R_2$ are independently selected from the group consisting of hydrogen atoms, aromatic groups (such as a phenyl or tolyl group) and alkyl groups. In an embodiment, at least one of $R_1$ and $R_2$ is an alkyl group. Such an amino group is an alkylamino group. In an embodiment, $R_1$ and $R_2$ are both alkyl groups. Such an amino group is a dialkylamino group.

An "activated ester" is an ester with a good leaving group, such as a N-hydroxysuccinimide ester, a N-hydrophthalimide ester, a perfluorinated ester or an acylurea. An example of perfluorinated ester is the following:

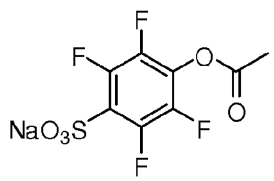

According to the present description, the optionally substituted aromatic group Ar is selected from the group consisting of:

- a phenyl group;
- a heteroaromatic group, such as pyridine, thiophene, imidazole, thiazole, pyrazole, pyrole or furane; and
- a polyaromatic group, such as anthracene or phenanthrene.

The phenyl, heteroaromatic and/or polyaromatic group may be substituted with at least one substituent (in addition to the F group when the aromatic is substituted with a F group), preferably selected from the group consisting of a halogen atom, an alkyl group, an alkoxy group, a carboxyl COOH group, a $COOR_3$ group, wherein $R_3$ is an alkyl group, and a nitro $NO_2$ group.

In an embodiment, the optionally substituted aromatic group is an optionally substituted phenyl group, preferably a non-substituted phenyl group.

The linker optionally comprised in R may be any hydrocarbon chain comprising C and H atoms and optionally O and/or N and/or S atoms, such as a $-(CH_2)q(CH_2O)r$ $-COO-(CH_2)q'(CH_2O)r'$ group, a $-(CH_2)q(CH_2O)r-NH-C(=O)-(CH_2)q'(CH_2O)r'$ group, a $-(CH_2)q(CH_2O)r-CONH-(CH_2)q'(CH_2O)r'$ group, wherein q and r are integers independently ranging from 0 to 10, or a triazolyl group, for instance.

In an embodiment of the description, X is a halogen atom.

When Ar is an optionally substituted phenyl group, the functional group F is in *meta* or *para* position of the phenyl group, preferably in *para* position.

When Ar is an optionally substituted heteroaromatic or polyaromatic group, the functional group F can be in any position of the aromatic group.

In a preferred embodiment of the description, the functional group F is selected from the group consisting of:

- a carboxylic acid COOH group,
- a thiol SH group,
- a maleimide

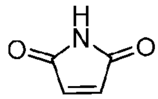

group,
- an activated ester,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- an isocyanate (-N=C=O) or isothiocyanate (-N=C=S) group,
- a chlorosulfonyl ($-SO_2Cl$) group,
- a -C≡C-C≡N group, and
- an alkyl group substituted by at least one of the groups above.

In a highly preferred embodiment of the description, the functional group F is selected from the group consisting of a carboxylic acid COOH group, an activated ester, such as a N-hydroxysuccinimide, a N-hydrophthalimide ester, a perfluorinated ester or an acylurea, and an alkyl group substituted by at least one of these groups.

In a preferred embodiment, F' is a C=O group and R is an amino group. Preferably, the R amino group is bonded through the nitrogen atom to the C=O F' group, thus forming a urea moiety. In another preferred embodiment, F' is a C=O group and R is an alkoxy group such as an tert-butoxy group. Preferably, the R alkoxy group is bonded through the oxygen atom to the C=O F' group, thus forming a carbamate moiety.

In a preferred embodiment, F' is a C=O group, R is an amino or alkoxy group forming a urea or carbamate moiety respectively as disclosed above, and X is a halogen atom, preferably selected from bromine and chlorine atoms.

Examples of iminosydnones of formula (I) that may be used in the above process are selected from the group consisting of:

- (2-(4-hydroxyphenyl)-1,2,3-oxadiazol-2-ium-5-yl)(p-tolylcarbamoyl)amide ;
- (2-(4-carboxyphenyl)-1,2,3-oxadiazol-2-ium-5-yl)(p-tolylcarbamoyl)amide ; and
- ((4-(ethoxycarbonyl)phenyl)carbamoyl)(2-(4-hydroxyphenyl)-1,2,3-oxadiazol-2-ium-5-yl)amide.

In an embodiment, n is from 1 to 50, preferably from 1 to 30. In a specific embodiment, n is 1.

The contacting step of the processes according to the invention may be performed by any technique known in the art, for instance by dissolution and/or dispersion of both compounds in a solvent. The contacting may be performed under stirring, for instance mechanical or magnetic stirring. The contacting may be performed at room temperature, *ie* at a temperature comprised between 18 and 25°C, or at low temperature, *ie* at a temperature inferior to 18°C, or even at high temperature, *ie* at a temperature higher than 25°C, for instance comprised between 25 and 150°C.

The process of preparation of a functionalized compound according to the invention may comprise a preliminary step of covalent bonding of a reactive group to C1, in the case where C1 does not bear a reactive group able to react with the functional group F of the iminosydnone compound. However, in a preferred embodiment, C1 intrinsically bears such a reactive group. In an embodiment, the reactive group is selected from the group consisting of:

- a carboxylic acid COOH group,
- a thiol SH group,
- a maleimide

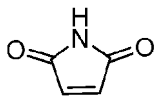

group,

- an activated ester
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR")_2$ group, wherein R" is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR''' group, wherein R''' is an alkyl group.

In a preferred embodiment, the reactive group is selected from the group consisting of:

- a carboxylic acid COOH group,
- a thiol SH group,
- a maleimide

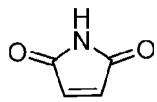

group,

- an activated ester,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- an isocyanate (-N=C=O) or isothiocyanate (-N=C=S) group,
- a chlorosulfonyl ($-SO_2Cl$) group, and
- a -C≡C-C≡N group.

In a highly preferred embodiment, the reactive group is an amino group or a thiol group.

The racemic forms, tautomers, enantiomers, diastereoisomers, epimers, solvates and salts of the compounds used in the process according to the invention are also part of the scope of the present disclosure.

As examples of salts may be cited acid addition salts, such as hydrochloric, hydrobromic, hydroiodic, phosphoric, and the like. Representative examples of suitable organic acids include formic, acetic, trichloroacetic, trifluoroacetic, propionic, benzoic, cinnamic, citric, fumaric, maleic, methanesulfonic and the like. Further examples of pharmaceutically acceptable inorganic or organic acid addition salts include the pharmaceutically acceptable salts listed in J. Pharm. Sci. 1977, 66, 2, and in Handbook of Pharmaceutical Salts: Properties, Selection, and Use edited by P. Heinrich Stahl and Camille G. Wermuth 2002.

Another object of the description is a functionalized compound of interest C1 of formula (II):

(II) 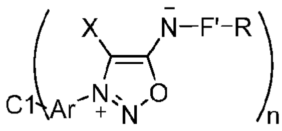

wherein C1, Ar, X, F', n and R are as defined above and C1 and Ar are covalently linked by a functional group. This functional group results from the reaction between the reactive group of C1 and the functional group F, such as an ester or amide function.

Another object of the invention is a conjugate of formula (IV):

(IV) 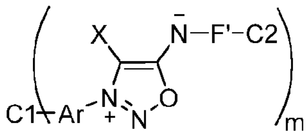 ,

wherein X, Ar, F' and C1 are as defined in claim 1. m is an integer which ranges from 1 to 100, C2 is a compound of interest selected from an antibody, a protein, a drug, a fluorophore, a nanoparticle and a polymer, C1 and Ar are covalently linked by a functional group (resulting from the reaction between the reactive group of C1 and the functional group F) and C2 and F' are covalently linked by a functional group. This functional group results from the reaction between the reactive group of C2 and R and may be for instance as an ester or amide function. In an embodiment, m is from 1 to 50, preferably from 1 to 30. In a specific embodiment, m is 1.

Another object of the description is a process for the preparation of a conjugate of formula (IV) as described above, comprising the step of contacting a compound of formula (II) with a compound of interest C2 bearing a reactive group which is able to react with R. It is understood that, in this embodiment of the invention, R is itself a reactive group.

Specific examples of R for use in this embodiment are:

- a carboxylic acid COOH group,
- a thiol SH group,
- a maleimide

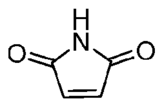

group,
- an activated ester
- an amino (-NRR') group, wherein R and R' are independently hydrogen atoms, alkyl, alkene, alkyne or aryl groups as defined above, and
- a -C≡C-C≡N group.

The process of preparation of the above conjugate may comprise a preliminary step of covalent bonding of a reactive group to C2, in the case where C2 does not bear a reactive group able to react with the reactive group R of the iminosydnone compound. However, in a preferred embodiment, C2 intrinsically bears such a reactive group.

In an embodiment of the description, this reactive group of C2 is selected from the group consisting of :

- a carboxylic acid COOH group,
- a thiol SH group,
- a maleimide

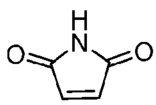

group,

- an activated ester
- a halogen atom,
- an alkene or alkyne group, optionally interrupted by at least one heteroatom selected among O, N and S,
- an amino (-NRR’) group, wherein R and R’ are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR'')_2$ group, wherein R" is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR’’’ group, wherein R’’’ is an alkyl group.

In a preferred embodiment, the reactive group is selected from the group consisting of:

- a carboxylic acid COOH group,
- a thiol SH group,
- a maleimide

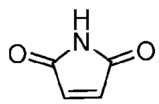

group,
- an activated ester,
- an amino (-NRR’) group, wherein R and R’ are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- an isocyanate (-N=C=O) or isothiocyanate (-N=C=S) group,
- a chlorosulfonyl ($-SO_2Cl$) group, and
- a -C≡C-C≡N group.

In a highly preferred embodiment of the description, the reactive group is an amino group or a thiol group.
Another object of the invention is a process for the preparation of a conjugate of formula (V):

(V) 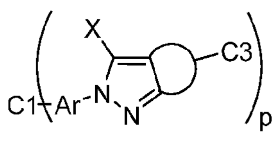,

wherein p is an integer ranging from 1 to 100, C1, Ar and X are as defined in claim 1, C1 is a first compound of interest, and C3 is a third compound of interest selected from an antibody, a protein, a drug, a fluorophore, a nanoparticle and a polymer, comprising a step of contacting a compound of formula (VI):

(VI) 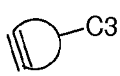,

with a conjugate of formula (IV) according to the invention. In an embodiment, p is from 1 to 50, preferably from 1 to 30.

In a specific embodiment, p is 1. This process simultaneously releases a derivative of C2 of formula (VII):

(VII) 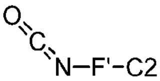.

The process for the preparation of a conjugate of formula (V) may comprise a preliminary step of covalent bonding of a strained alkyne moiety to the compound of interest C3.

By "strained alkyne", it is meant a cyclic alkyne, in particular cyclooctynes such as bicyclo-[6.1.0]-nonyne (BCN) or cycloheptynes such as tetramethylthiacycloheptyne (TMTH). In the case of BCN, for instance, the compound of interest C3 may be bonded to BCN by reacting a C3-O-CO-Cl compound with the hydroxy group of BCN, so as to form a carbamate bond. In the case of TMTH, the latter may be alkylated on its sulphur atom by reaction with a C3-CH2-Br compound.

Another object of the description is a conjugate of formula (V):

(V) 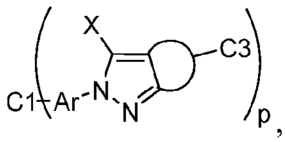,

wherein C1, Ar, X and C3 are as defined above.

Another object of the description is a process for releasing a derivative of C2 of formula (VII):

(VII) 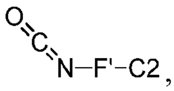,

comprising a step of contacting a conjugate of formula (IV):

(IV) 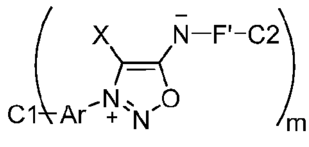

as described above with a compound comprising a strained alkyne moiety, preferably a cyclic alkyne moiety, in particular a cyclooctyne moiety, for instance BCN (bicyclononyne). More preferably, the conjugate of formula (IV) is reacted with a compound of formula (VI) as described above.

Another object of the description is an iminosydnone of formula (I'): wherein

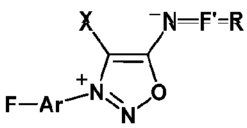 (I'),

X is a halogen atom,
F, F', R and Ar are as described above.

The iminosydnone compounds of formulas (I) and (I') can be used advantageously in order to couple both compounds of interest C1 and C3 or C1 and C2 and/or release the compound of interest C2, as explained above.

In an embodiment, C1 is a nanoparticle or an antibody, C2 is a drug and C3 is a fluorophore. In this embodiment, the iminosydnone of the invention affords the release of the drug, which may then enter its target cells, and the labeling of the nanoparticle or the antibody. In a particular embodiment, C1 is an antibody and this embodiment leads to the release of a drug from a therapeutic antibody and at the same time the labeling of the antibody, therefore allowing theranostic applications. In an especially preferred embodiment of this invention, the drug is a chemotherapeutic drug

and the labeling of the antibody allows imaging the tumor.

The iminosydnones of formulas (I) and (I') react very efficiently with strained alkynes, such as cyclic alkynes, in particular cyclooctynes. The coupling reaction implies the formation of a cycloadduct formed by a [3+2] cyclization turning to a stable pyrazole said cycloadduct by retro-Diels Alder reaction, triggering the release of a R-F'-NCO molecule, which in aqueous medium, for instance in water, may spontaneously evolve to R-F'-$NH_2$ through hydrolysis and/or decarboxylation reactions. The reaction with a compound according to the invention with a cycloalkyne is represented as example on the scheme below.

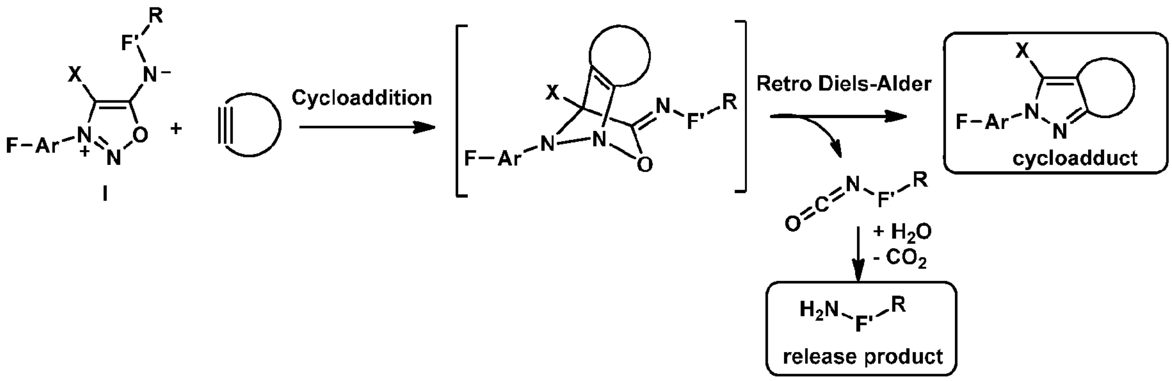


The present compounds allow the efficiency of the reaction to be maintained in biological media, such as culture media, cell lysates or plasma.

These iminosydnones may be synthesized by any appropriate method known by one of ordinary skill in the art.

For instance, the scheme below presents different synthesis routes for iminosydnones of formulas (I) and (I'). The Ar groups substituting the nitrogen atom of the iminosydnones may be substituted with a F group. In this scheme, the term NBS designates N-bromosuccinimide.

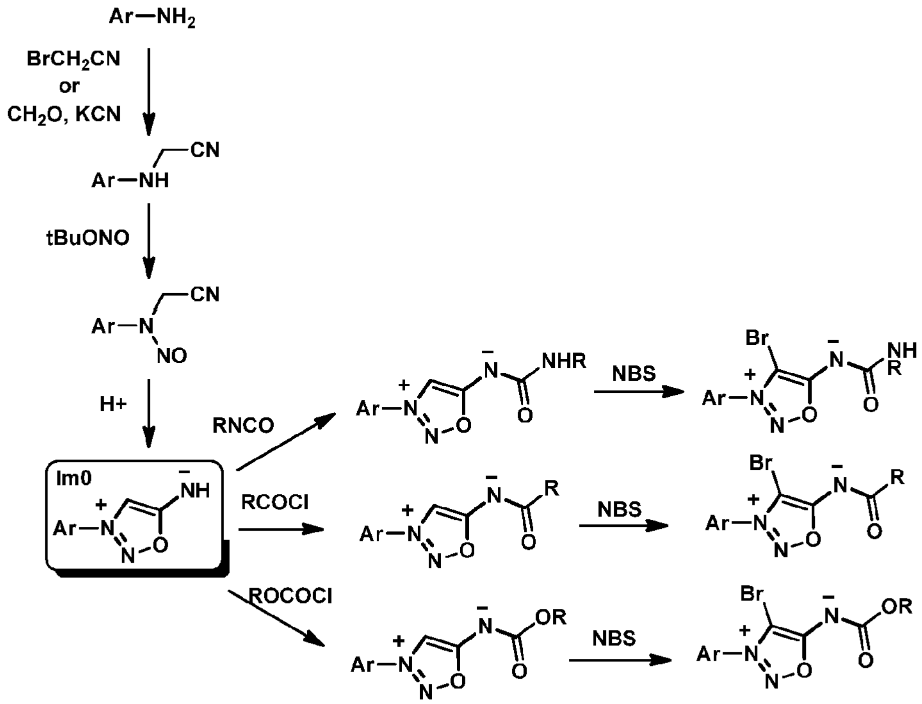


The following examples are provided as illustrative. They are not covered by the claims and, therefore, represent reference examples.

## Examples

### Example 1: Synthesis of sydnones

In the following examples, the provided yields are molar yields unless specified differently.

**Im0-1 (intermediate): 5-amino-3-(4-hydroxyphenyl)-1,2,3-oxadiazol-3-ium chloride**

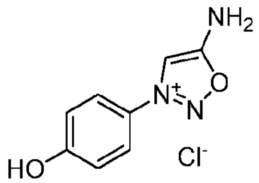

To a solution of *2-((4-hydroxyphenyl)amino)acetonitrile* (2.56 g, 12 mmol) in tetrahydrofuran THF (60 mL) was added amyl nitrite (1.85 eq, 22.2 mmol). The mixture was stirred for 16h at room temperature (r.t.) and then HCl (5 mL, 4M solution in dioxane) was added. The resulting mixture was stirred for 24 h at r.t. The precipitate was collected by filtration and washed with $Et_2O$ and dried to yield pure product as a yellow solid (850 mg, 4.8 mmol, 40%).

**$^1$H NMR (400 MHz, DMSO-$d_6$, δ ppm):** 9.71 (s, 2 H), 8.48 (s, 1 H), 7.88 (d, *J* = 9.1 Hz, 2 H), 7.09 (d, *J* = 9.1 Hz, 2 H). **$^{13}$C NMR (100 MHz, DMSO-$d_6$, δ ppm):** 169.1, 162.1, 124.1, 124.0, 116.6, 101.0.

**Im0-2 (intermediate): 5-amino-3-(4-carboxyphenyl)-1,2,3-oxadiazol-3-ium chloride**

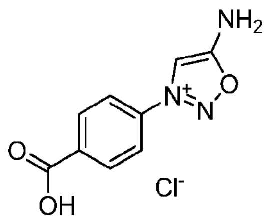

To a solution of *4-((cyanomethyl)amino)benzoic acid* (1.13 g, 6.42 mmol) in THF (60 mL) was added amyl nitrite (1.85 eq, 11.9 mmol). The mixture was stirred for 16h at r.t. and then HCl (5 mL, 4M solution in dioxane) was added. The resulting mixture was stirred for 24 h at r.t. The precipitate was collected by filtration and washed with $Et_2O$ and dried to yield pure product as a white solid (161 mg, 0.67 mmol, 10%).
**$^1$H NMR (400 MHz, DMSO-$d_6$, δ ppm):** 9.97 (br. s., 2 H), 8.73 (s, 1 H), 8.27 (d, *J* = 8.6 Hz, 2 H), 8.18 (d, *J* = 8.6 Hz, 2 H).
**$^{13}$C NMR (100 MHz, DMSO-$d_6$, δ ppm):** 169.4, 165.7, 135.5, 135.1, 131.0, 123.1, 102.7.

**Im 6 (comparative): (2-(4-hydroxyphenyl)-1,2,3-oxadiazol-2-ium-5-yl)(p-tolylcarbamoyl)amide**

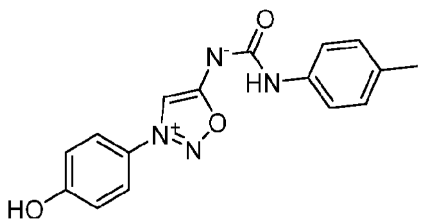

To a solution of *p-tolyl isocyanate* (47 mg, 0.351 mmol) and iminosydnone **Im0-1 S** (75 mg, 0.351 mmol) in THF (5 mL) was added a solution of $NaHCO_3$ (1 eq., 30 mg, 0.35 mmol) in $H_2O$ (1 mL). The resulting solution was stirred at room temperature for 14 hours. The organic layer was separated, dried over $MgSO_4$ and evaporated. The residue

was purified by semi-preparative HPLC (MeCN/$H_2O$ gradient) to afford **Im6** as a white solid (18.3 mg, 0.059 mmol, 17%).
**$^1$H NMR (400 MHz, DMSO-$d_6$, δ ppm):** 9.29 (br. s., 1 H), 8.38 (s, 1 H), 7.89 (d, *J* = 9.0 Hz, 2 H), 7.54 (d, *J* = 8.2 Hz, 2 H), 7.07 - 6.93 (m, *J* = 6.5, 8.5 Hz, 4 H), 2.22 (s, 3 H).
**$^{13}$C NMR (100 MHz, DMSO-$d_6$, δ ppm):** 172.2, 161.1, 158.8, 138.5, 129.9, 128.8, 125.3, 123.7, 117.9, 116.4, 102.1, 20.4.

**Im 8 (comparative): ((4-(ethoxycarbonyl)phenyl)carbamoyl)(2-(4-hydroxyphenyl)-1,2,3-oxadiazol-2-ium-5-yl)amide.**

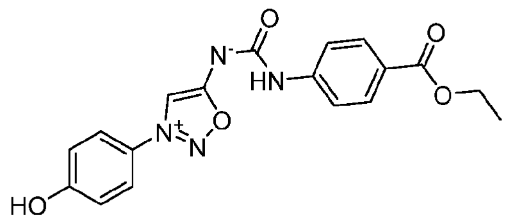

To a solution of *ethyl 4-isocyanatobenzoate* (67 mg, 0.351 mmol) and iminosydnone **Im0-1** (75 mg, 0.351 mmol) in THF (5 mL) was added a solution of $NaHCO_3$ (1 eq., 30 mg, 0.35 mmol) in $H_2O$ (1 mL). The resulting solution was stirred at room temperature for 14 hours. The organic layer was separated, dried over $MgSO_4$ and evaporated. The residue was purified by semi-preparative HPLC (MeCN/$H_2O$ gradient) to afford **Im 8** as a yellow solid (16.1 mg, 0.044 mmol, 13%).
**$^1$H NMR (400 MHz, DMSO-$d_6$, δ ppm):** 10.60 (br. s., 1 H), 9.82 (s, 1 H), 8.49 (s, 1 H), 7.91 (d, *J* = 9.0 Hz, 2 H), 7.84 (d, *J* = 8.8 Hz, 2 H), 7.77 (d, *J* = 8.8 Hz, 2 H), 7.02 (d, *J* = 9.0 Hz, 2 H), 4.27 (q, *J* = 7.0 Hz, 2 H), 1.30 (t, *J* = 7.0 Hz, 3 H).
**$^{13}$C NMR (100 MHz, DMSO-$d_6$, δ ppm):** 172.4, 165.5, 161.1, 158.6, 145.6, 130.0, 125.2, 123.7, 122.1, 117.0, 116.3, 102.6, 60.0, 14.2.
**MS (ESI) *m/z*:** 369.2 [M+H]$^+$.

**Im 7: (2-(4-carboxyphenyl)-1,2,3-oxadiazol-2-ium-5-yl)(p-tolylcarbamoyl)amide**

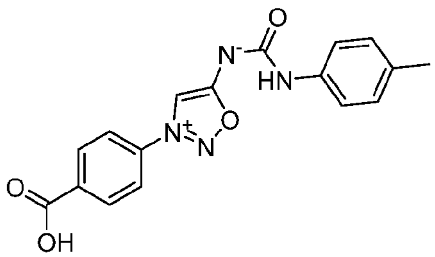

To a solution of *p-tolyl isocyanate* (15 mg, 0.113 mmol) and iminosydnone **Im0-2** (27 mg, 0.113 mmol) in THF (5 mL) was added a solution of $NaHCO_3$ (1 eq., 10 mg, 0.12 mmol) in $H_2O$ (1 mL). The resulting solution was stirred at room temperature for 14 hours. The organic layer was separated, dried over $MgSO_4$ and evaporated. The residue was purified by semi-preparative HPLC (MeCN/$H_2O$ gradient) to afford **Im 7** as a yellow solid (8.6 mg, 0.025 mmol, 22%).
**$^1$H NMR (400 MHz, DMSO-$d_6$, δ ppm):** 9.41 (br. s., 1 H), 8.69 (s, 1 H), 8.21 (s, 4 H), 7.53 (d, *J* = 8.1 Hz, 2 H), 7.04 (d, *J* = 8.1 Hz, 2 H), 2.22 (s, 3 H).
**$^{13}$C NMR (100 MHz, DMSO-$d_6$, δ ppm):** 171.8, 165.9, 157.9, 138.1, 136.5, 134.5, 131.0, 130.2, 128.8, 122.6, 118.1, 103.5,20.3.
**MS (ESI) *m/z*:** 339.1 [M+H]$^+$.

**Im13: (3-(4-carboxyphenyl)-1,2,3-oxadiazol-3-ium-5-yl)((2-(3-(2,5-dioxo-2,5-dihydro-1H-pyrrol-1-yl)propanamido)ethyl)carbamoyl)amide**

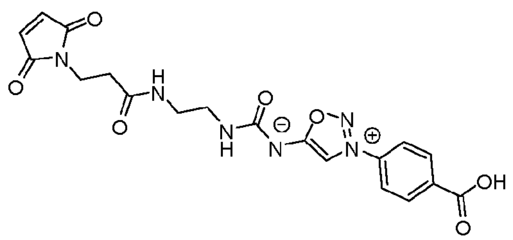

The following synthesis process was conducted to prepare the above compound, referred to as K:

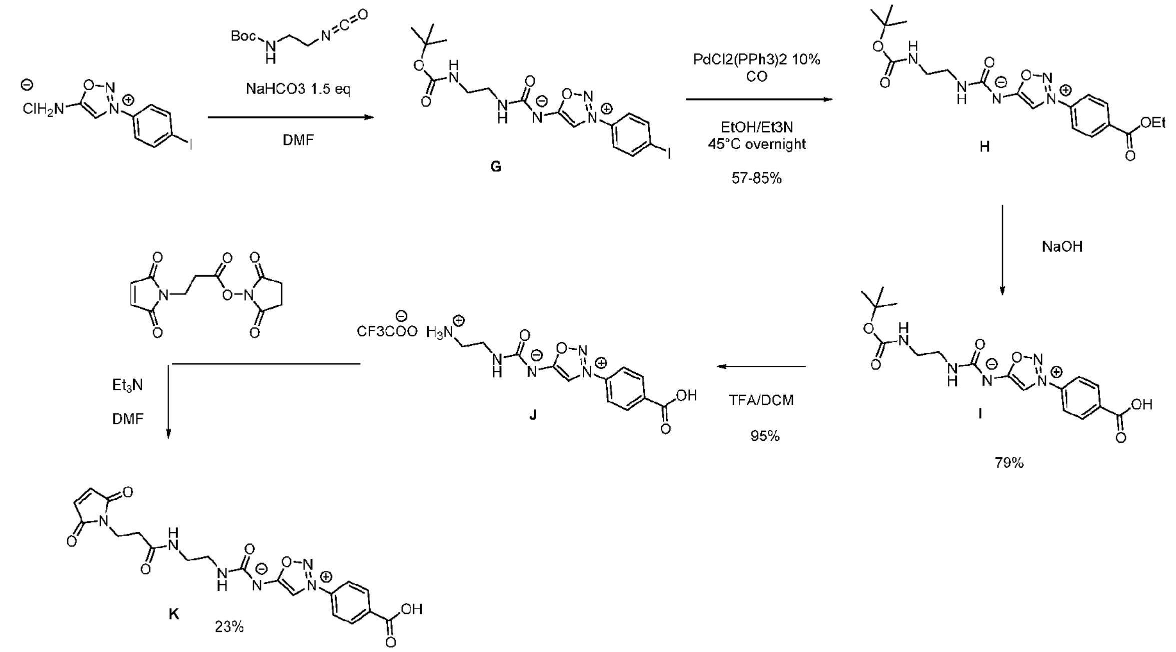


Step (a): synthesis of G - **((2-((tert-butoxycarbonyl)amino)ethyl)carbamoyl)(3-(4-iodophenyl)-1,2,3-oxadiazol-3-ium-5-yl)amide**

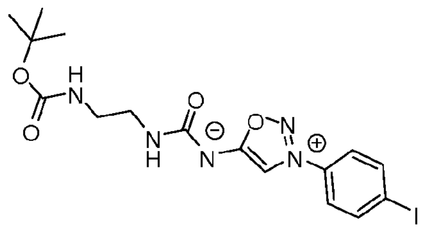

To a suspension of 5-amino-3-(4-iodophenyl)-1,2,3-oxadiazol-3-ium chloride and $NaHCO_3$ in dry DMF (1 mL) was added the tert-butyl (2-isocyanatoethyl)carbamate. The mixture stirred at room temperature overnight. The mixture was quenched with NH4Cl (15 mL) then extracted with AcOEt. The combined organic layers were dried (MgSO4) and concentrated under reduced pressure. The crude material was though flash chromatography.

**$^1$H NMR** ($CDCl_3$, 400MHz, ppm): $\delta$ = 8.14 (s, 1H), 7.98 (d, J =8.4 Hz, 2H), 7.52 (d, J = 8.4 Hz, 2H), 5.77 (bs, 1H), 5.07 (bs, 1H), 3.40-3.36 (m, 2H), 3.30-3.26 (m, 2H), 1.42 (s, 9H);

**$^{13}$C NMR** ($CDCl_3$, 101MHz, ppm): $\delta$ = 172.6, 161.9, 156.1, 139.6 (2C), 133.5, 122.7 (2C), 101.7, 99.2, 79.2, 41.0, 40.0, 28.3 (3C) ;

**IR (v, cm$^{-1}$)**: 3283, 2976, 1691, 1599, 1632, 1515, 1430, 1365, 1274, 1220, 1007, 959;

**MS** (ESI) *m/z* : $[M+H]^+$ = 474.2.

Step (b): synthesis of H - **((2-((tert-butoxycarbonyl)amino)ethyl)carbamoyl)(3-(4-(ethoxycarbonyl)phenyl)-1,2,3-oxadiazol-3-ium-5-yl)amide**

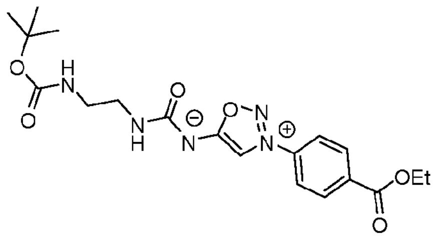

((2-((tert-butoxycarbonyl)amino)ethyl)carbamoyl)(3-(4-iodophenyl)-1,2,3-oxadiazol-3-ium-5-yl)amide (45 mg, 0.095 mmol, 1 equiv.) was dissolved in a mixture of absolute EtOH (1 mL) and triethylamine (1 mL). [$PdCl_2(PPh_3)_2$] (6.3 mg, 0.009 mmol, 10 mol%) was added and the reaction mixture was kept under a CO atmosphere for 15 h and heated at 45 °C. The resulting suspension was cooled to room temperature and filtred through Celite eluting with ethyl acetate, and the inorganic salts were removed. The filtrate was concentrated and purification of the residue by silica gel column chromatography gave the desired product.

**$^1$H NMR** ($CDCl_3$, 400MHz, ppm): δ = 8.31 (d, J = 8.6 Hz, 2H), 8.20 (s, 1H), 7.88 (d, J = 8.6 Hz, 2H), 5.76 (bs, 1H), 5.03 (bs, 1H), 4.45 (q, J = 7.1 Hz, 2H), 3.40-3.36 (m, 2H), 3.30-3.25 (m, 2H), 1.45-1.41 (m, 12H);
**$^{13}$C NMR** ($CDCl_3$, 101MHz, ppm): δ = 172.5, 164.4, 161.7, 156.1, 136.7, 134.5, 131.5 (2C), 121.4 (2C), 102.1, 79.1, 61.9, 41.0, 40.4, 28.3 (2C), 14.2 ;
**IR (v, cm$^{-1}$)**: 3288, 1978, 1713, 1635, 1604, 1512, 1441, 1366, 1274, 1173, 1108, 958, 770; **MS** (ESI) *m/z* : $[M+H]^+$ = 420.1 ; **Mp:** 139-141 °C.

Step (c): synthesis of I - **((2-((tert-butoxycarbonyl)amino)ethyl)carbamoyl)(3-(4-carboxyphenyl)-1,2,3-oxadiazol-3-ium-5-yl)amide**

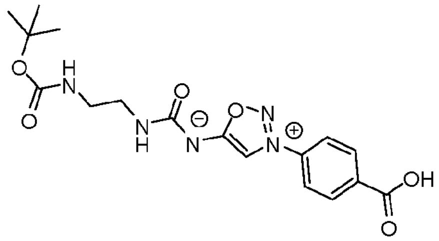

To a solution of ((2-((tert-butoxycarbonyl)amino)ethyl)carbamoyl)(3-(4-(ethoxycarbonyl)phenyl)-1,2,3-oxadiazol-3-ium-5-yl)amide (65 mg, 0.155 mmol) in EtOH (1 mL) /THF (1 mL) was added NaOH (5 eq). The resulting suspension was stirred at room temperature. After 2h, the mixture was diluted with water then extracted with AcOEt (3 times). The aqueous phase was acidified with HCl (2 N) until pH ca. 2. Then it was extracted with AcOEt (3 times). The combined organic layers were dried (MgSO4) and concentrated under reduced pressure to give the desired product.

**$^1$H NMR** (DMSO, 400MHz, ppm): δ = 9.35 (s, 1H), 8.10 (d, J = 8.4 Hz, 2H), 7.99 (d, J = 8.4 Hz, 2H), 6.94 (t, J = 5.2 Hz, 1H),;
**$^{13}$C NMR** (DMSO, 101MHz, ppm): δ = 172.2, 167.7, 161.3, 156.0, 141.3, 135.2, 131.0 (2C), 121.9 (2C), 102.6, 78.0, 40.5, 39.2, 9.6 (3C);
**MS** (ESI) *m/z* : $[M+H]^+$ = 392.3.

Step (d): synthesis if J - **((2-ammonioethyl)carbamoyl)(3-(4-carboxyphenyl)-1,2,3-oxadiazol-3-ium-5-yl)amide 2,2,2-trifluoroacetate**

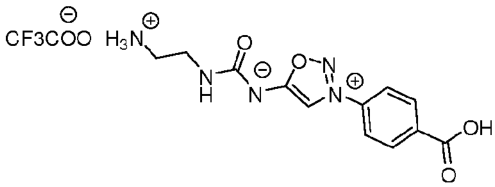

To a solution of ((2-((tert-butoxycarbonyl)amino)ethyl)carbamoyl)(3-(4-carboxyphenyl)-1,2,3-oxadiazol-3-ium-5-yl)amide (1 equiv.) in DCM (2 mL) was added TFA (0.5 mL). The resulting solution was stirred at room temperature. After 4h, the solvent was evaporated under reduced pressure (with re-solubilisation in MeOH and re-evaporation 2 times in order to achieve full elimination of TFA).

$^1$**H NMR** (MeOD, 400MHz, ppm): $\delta$ = 9.21 (s, 1H), 8.37 (d, J = 8.4 Hz, 2H), 8.20 (d, J = 8.4 Hz, 2H), 3.20-3.10 (m, 4H);
**MS** (ESI) *m/z* : $[M+H]^+$ = 292.1.

Step (e): synthesis of K = **Im13**

To a homogeneous solution of ((2-ammonioethyl)carbamoyl)(3-(4-carboxyphenyl)-1,2,3-oxadiazol-3-ium-5-yl)amide 2,2,2-trifluoroacetate (1 equiv.) in dry DMF (1 mL) was added TEA (3 equiv.). The mixture was cooled at 0 °C and then 2,5-dioxopyrrolidin-1-yl 3-(2,5-dioxo-2,5-dihydro-1H-pyrrol-1-yl)propanoate (1 equiv.) was added to the mixture. After 18h, the mixture was concentrated under vacuum. The residue was dissolved in EtOAc (100 mL) and extracted with 0.1 N HCl (2×). The organic layer was washed with brine (2×) and dried (Na2SO4) and concentrated to give the desired product.

$^1$**H NMR** (MeOD, 400MHz, ppm): $\delta$ = 9.27 (s, 1H), 8.39 (d, J = 8.6 Hz, 2H), 8.23 (d, J = 5.6 Hz, 2H), 6.81 (s, 2H), 3.78 (t, J = 6.7 Hz, 2H), 3.37-3.32 (m, 4H), 2.46 (t, J = 6.7 Hz, 2H); **MS** (ESI) *m/z* : $[M+H]^+$ = 443.1.

**Im14**: **((4-(cyanoethynyl)phenyl)carbamoyl)(3-(4-(ethoxycarbonyl)phenyl)-1,2,3-oxadiazol-3-ium-5-yl)amid**

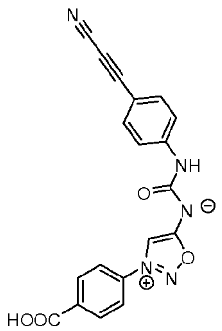

This compound was prepared according to a process comprising the following successive steps.

*Step 1: Synthesis of* ***2-((4-iodophenyl)amino)acetonitrile***

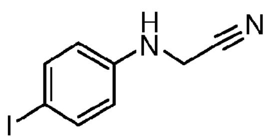

To a 250 mL round bottom flask was charged with 4-iodoaniline (5 g, 22.83 mmol, 1 equiv.), NaI (3.42 g, 22.83 mmol, 1 equiv.), $K_2CO_3$ (3.7 g, 27.39 mmol, 1.2 equiv.) and MeCN (60 mL) was added dropwise chloroacetonitrile (2.86 mL, 45.65 mmol, 2 equiv.) the mixture was stirred at reflux under nitrogen atmosphere.

After 48h, the mixture was allowed to cool at room temperature and filtered through a fritted glass filter with AcOEt (200 mL). The solution was then washed with $NaCl_{sat}$ (100 mL). The combined organic layers were dried (MgSO4) and concentrated under reduced pressure. The resulting material was purified though flash chromatography (100% cyclohexane to 80% cyclohex : 20% AcOEt) to afford 5.46 g (21.16 mmol, 92% yield) of analytically pure compound.

**$^1$H NMR** ($CDCl_3$, 400MHz, ppm): δ = 7.52 (d, *J* = 8.8 Hz, 2H), 6.48 (d, *J* = 8.8 Hz, 2H), 4.07 (s, 2H); **$^{13}$C NMR** ($CDCl_3$, 101MHz, ppm): δ = 144.6, 138.2 (2H), 116.5, 115.7 (2H), 81.5, 32.4; **MS** (ESI) *m/z* : $[M+H]^+$ = 259.0 ;

*Step 2: Synthesis of* ***5-amino-3-(4-iodophenyl)-1,2,3-oxadiazol-3-ium chloride***

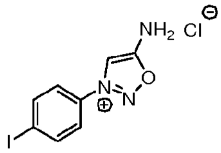

To a solution of 2-((4-iodophenyl)amino)acetonitrile (3.5 g, 13.56 mmol, 1 equiv.) in THF (20 mL) stirred at room temperature was added dropwise isopentyl nitrite (5.5 mL, 40.68 mmol, 3 equiv.). The mixture was stirred until full conversion to the corresponding nitroso aniline was achieved (TLC monitoring). Afterword, the solvent was evaporated to dryness under high vacuum to eliminate the excess of nitrite. Then the residue was dissolved in dry THF (10 mL) and a solution of dry HCl in dioxane 4 M (34 mL, 10 equiv.) was added. The mixture was stirred at room temperature. After 16h, the precipitate formed was filtered and washed with $Et_2O$ (150 mL) to afford analytically pure product.

**$^1$H NMR** (DMSO, 400MHz, ppm): δ = 10.10 (s, 2H), 8.69 (s, 1H), 8.14 (d, *J* = 8.4 Hz, 2H), 7.84 (d, *J* = 8.4 Hz, 2H); **$^{13}$C NMR** (DMSO, 101MHz, ppm): δ = 169.4, 139.1 (2C), 132.4, 124.4 (2C), 102.3, 101.5; **MS** (ESI) *m/z* : $[M-Cl]^+$ = 288.2.

*Step3: Synthesis of* ***(tert-butoxycarbonyl)(3-(4-iodophenyl)-1,2,3-oxadiazol-3-ium-5-yl)amide***

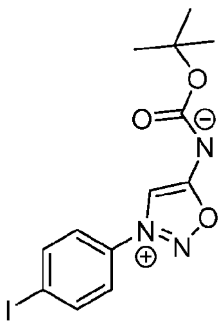

To a solution of 5-amino-3-(4-iodophenyl)-1,2,3-oxadiazol-3-ium chloride (4.08 mmol, 1 equiv.), $NaHCO_3$ (6.12 mmol, 1.5 equiv.) and DMAP (0.41 mmol, 10 mol%) and THF (30 mL) was added dropwise di-tert-butyl dicarbonate (6.12 mmol, 1.5 equiv.). The resulting mixture was stirred at room temperature for 48h. The heterogeneous mixture was quenched with $NH_4Cl$ (35 mL) and extracted with AcOEt (3* 30 mL). The combined organic layers were dried over $MgSO_4$ and concentrated under reduced pressure. The crude material was purified though automated flash chromatography (from 100% cyclohexane to 80% cyclohexane / 20% AcOEt) to afford 1.36 g (3.51 mmol, 86% yield) of analytically pure compound.

**$^1$H NMR** ($CDCl_3$, 400MHz, ppm): δ = 8.10 (s, 1H), 7.98, (d, *J* = 8.7 Hz, 2H), 7.52 (d, *J* = 8.7, 2H) 1.51 (s, 9H); **$^{13}$C NMR** ($CDCl_3$, 101MHz, ppm): δ = 174.3, 160.2, 139.7 (2C), 133.3, 122.7 (2C), 102.3, 99.48, 79.4, 28.1 (3C);
**IR (v, cm$^{-1}$)**: 3170, 3092, 2973, 1783, 1752, 1660, 1596, 1580, 1488, 1364, 1291, 1048, 1005, 964, 826, 728;
**MS** (ESI) *m/z* : [M+H] + = 388.1 ;
**Mp:** 134-136 °C.

*Step 4: Synthesis of* ***(tert-butoxycarbonyl)(3-(4-(ethoxycarbonyl)phenyl)-1,2,3-oxadiazol-3-ium-5-yl)amide***

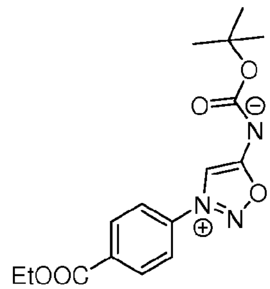

(tert-butoxycarbonyl)(3-(4-iodophenyl)-1,2,3-oxadiazol-3-ium-5-yl)amide (300 mg, 0.774 mmol, 1 equiv.) was dissolved in a mixture of absolute EtOH (2.5 mL) and triethylamine (2.5 mL). [$PdCl_2(PPh_3)_2$] (81 mg, 0.116 mmol, 15 mol%) was added and the reaction mixture was kept under a CO atmosphere for 2.5 h and heated at 60 °C.
The resulting suspension was cooled to room temperature and filtred through Celite eluting with ethyl acetate, and the inorganic salts were removed. The filtrate was concentrated and purification of the residue by silica gel column chromatography gave the desired product. The resulting residue was purified by chromatography (from 100% cyclohexane to 70% cyclohexane / 30% AcOEt) to afford 183 mg (0.550 mmol, 71% yield) of analytically pure compound.

**$^1$H NMR** ($CDCl_3$, 400MHz, ppm): $\delta$ = 8.29 (d, *J* = 9.0 Hz, 2H), 8.16 (s, 1H), 7.87 (d, *J* = 9.0 Hz, 2H), 4.43 (q, *J* = 7.0 Hz, 2H), 1.51 (s, 9H), 1.42 (t, *J* = 7.0 Hz, 3H);

**$^{13}$C NMR** ($CDCl_3$, 101MHz, ppm): $\delta$ = 174.6, 164.3, 160.6, 136.6, 134.7, 131.6 (2C), 121.4 (2C), 102.5, 79.2, 62.0, 28.1 (3C), 14.2;
**IR (v, cm$^{-1}$)**: 3159, 2979, 1784, 1716, 1662, 1586, 1366, 1271, 1152, 1106, 1048, 1007, 963, 855, 769, 728, 689;
**MS** (ESI) *m/z* : $[M+H]^+$ = 334.3 ;
**Mp:** 151-153 °C.

*Step 5: Synthesis of* ***((4-(cyanoethynyl)phenyl)carbamoyl)(3-(4-(ethoxycarbonyl)phenyl)-1,2,3-oxadiazol-3-ium-5-yl)amide***

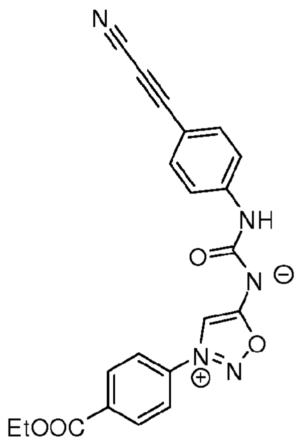

To a solution of (tert-butoxycarbonyl)(3-(4-(ethoxycarbonyl)phenyl)-1,2,3-oxadiazol-3-ium-5-yl)amide (91 mg, 0.273 mmol) in DCM (5 mL) was added TFA (0.5 mL). The mixture was stirred at room temperature for 3 hours. After it was reached full de-protection (monitoring by LCMS), the solvent was evaporated under reduced pressure (with re-solubilisation in MeOH and re-evaporation 2 times in order to achieve full elimination of TFA). In a separate flask, to a heterogeneous solution of triphosgene (0.33 equiv.) and 3-(4-aminophenyl)prop-2-ynenitrile (1 equiv.) in DCM (2 mL) was added, drop-wise at 0 °C, an aqueous solution of $NaHCO_3$ (3 equiv. in1.5 mL of water). The mixture was stirred at r.t. for 30 min, then the deprotected amine derivative was added in DCM. The mixture was stirred at room temperature. After 18h, the yellow heterogeneous mixture was diluted in Et2O (50 mL) and filtered. The yellow ppt was washed with Et2O, collected and dried under vacuum, to afford the desired product in 44 % yield.

**$^1$H NMR** (DMSO, 400MHz, ppm): $\delta$ = 9.98 (s, 1H), 8.72 (s, 1H), 8.23-8.22 (m, 4H), 7.77 (d, J = 8.5 Hz, 2H), 7.67 (d, J = 8.5 Hz, 2H), 4.37 (q, J = 7.0 Hz, 2 H), 1.34 (t, J = 7.0, 3H);
**IR (v, cm$^{-1}$)**: 3345, 3144, 2256, 1697, 1651, 1571, 1616, 1513, 1491, 1431, 1311, 1279, 1241, 1179, 1009, 931, 836, 535;

**MS** (ESI) *m/z* : $[M+H]^+$ = 402.1.

*Step 6: Synthesis of* ***Im14 - (3-(4-carboxyphenyl)-1,2,3-oxadiazol-3-ium-5-yl)((4-(cyanoethynyl)phenyl)carbamoyl)amide.***

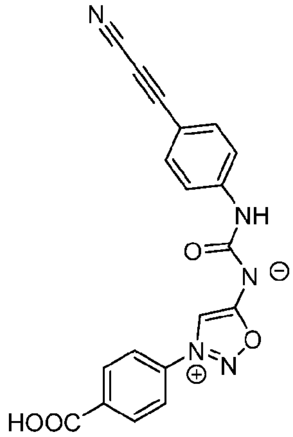

To a solution of ((4-(cyanoethynyl)phenyl)carbamoyl)(3-(4-(ethoxycarbonyl)phenyl)-1,2,3-oxadiazol-3-ium-5-yl)amide (55 mg, 0.15 mmol) in EtOH (1 mL) /THF (1 mL) was added NaOH (5 eq). The resulting suspension was stirred at room temperature. After 2h, the mixture was diluted with water then extracted with AcOEt (3 times). The aqueous phase was acidified with HCl (2 N) until pH ca. 2. Then it was extracted with AcOEt (3 times). The combined organic layers were dried (MgSO4) and concentrated under reduced pressure to give the desired product in 7% yield.

$^1$**H NMR** (DMSO, 400MHz, ppm): $\delta$ = 9.98 (s, 1H), 8.72 (s, 1H), 8.13-8.02 (m, 4H), 7.77 (d, J = 8.5 Hz, 2H), 7.67 (d, J = 8.5 Hz, 2H).

**MS** (ESI) *m/z* : $[M+H]^+$ = 374.2.

**Example 2: Kinetic study of the reaction of iminosydnones with cyclic alkynes**

**Experimental:**

Reactions of iminosydnones Im 2 and Im 5 as described in Table 1 below with tetramethylthiacycloheptyne (TMTH) was carried out in PBS buffer (0.1M, pH 7.4) at 100 $\mu$M concentration of iminosydnone and 150 $\mu$M concentration of cyclooctyne TMTH using the following procedure:

To 900 $\mu$L of PBS buffer were added 10 $\mu$L of the solution of benzamide (internal standard, 100 mM in DMSO), 1 $\mu$L of the solution of iminosydnone (100 mM in DMSO) and 1.5 $\mu$L of the solution of TMTH (100 mM in DMSO). The reaction mixture was injected in HPLC and the conversion was followed by measuring the normalized iminosydnone peak area.

**Results:**

Figures 1 and 2 present the HPLC monitoring of the reaction between the cyclic alkyne TMTH and iminosydnones Im5 and Im 2, respectively. The reaction has been conducted at 100 $\mu$M in PBS buffer (pH 7.4). The results indicated an almost complete reaction after 30 min proving that the reaction is fast enough to be useful for bioconjugation and release applications. The presence of a F group on the phenyl group would clearly not detrimentally affect these results. One can expect similar or even improved kinetics for the iminosydnones according to the invention.

**Example 3: Kinetic study of different iminosydnones**

The kinetics of the coupling reaction of different iminosydnones with two cyclic alkynes was studied, according to the following scheme.

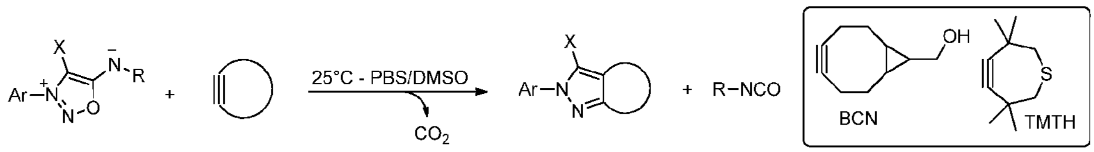


**Experimental:**

Reactions of iminosydnones with BCN (Biclyclononyne) or TMTH were carried out in PBS/DMSO (9:1) mixtures at 100 μM concentration of sydnones and 150 μM concentration of BCN or TMTH using the following procedure:
To 900 μL of phosphate buffered saline (PBS, 100 mM) was added 87.5 μL of DMSO, 10 μL of the solution of benzamide (internal standard, 100 mM in DMSO), 1 μL of the solution of iminosydnone (100 mM in DMSO) and 1.5 μL of the solution of BCN or TMTH (100 mM in DMSO). The reaction mixture was injected in HPLC every 30 min and the conversion was followed by measuring the normalized sydnone peak area.

**Results:**

Table 1 below presents the kinetic constant values K for these reactions, depending on the Ar group, the R group and the X group. Only iminosydnones 7, 13 and 14 are according to the invention.

| Comp. | Ar | F | X | R-F' | K (BCN) | K (TMTH) |
|---|---|---|---|---|---|---|
| **Im1** | $C_6H_5$ | - | H | H | no reaction | n.d. |
| **Im2** | $C_6H_5$ | - | H | $CH_3$-CO- | 0.002 | 2.2 |
| **Im3** | $C_6H_4$ | *p*CH3 | H | $C_6H_5CH_2$-CO- | 0.004 | n.d. |
| **Im4** | $C_6H_4$ | *p*CH3 | H | $C_6H_5CH_2$ $CH_2$-CO- | 0.034 | n.d. |
| **Im5** | $C_6H_5$ | - | H | *p*$CH_3C_6H_4$NH-CO- | 0.069 | 43.1 |
| **Im6** | $C_6H_4$ | *p*OH | H | *p*$CH_3C_6H_4$NH-CO- | 0.008 | 4.1 |
| **Im7** | $C_6H_4$ | *p*COOH | H | *p*$CH_3C_6H_4$NH-CO- | 0.108 | 61.2 |
| **Im8** | $C_6H_4$ | *p*OH | H | *p*$CO_2EtC_6H_4$NH-CO- | 0.008 | 2.5 |
| **Im9** | $C_6H_4$ | *p*CH3 | H | $C_6H_5CH_2$NH-CO- | 0.004 | n.d. |
| **Im10** | $C_6H_4$ | *p*CH3 | Br | $C_6H_5CH_2$NH-CO- | 0.060 | n.d. |
| **Im11** | $C_6H_4$ | *p*CH3 | H | $C_6H_5CH_2$O-CO- | 0.026 | n.d. |
| **Im12** | $C_6H_4$ | *p*CH3 | Br | $C_6H_5CH_2$O-CO- | 0.150 | n.d. |
| **Im13** | $C_6H_4$ | *p*COOH | H | Mal*-$(CH_2)_2$-CO-NH-$(CH_2)_2$- NH-CO- | n.d. | n.d. |
| **Im14** | $C_6H_4$ | *p*COOH | H | N≡C-C≡-$C_6H_4$-NH-CO | 0.132 | n.d. |

Mal*: maleimide

**Table 1.** K unit is $M^{-1}.sec^{-1}$, n.d. = not determined. Reactions were conducted with 100 μM of iminosydnones and 150 μM of BCN or TMTH in PBS 0.1 M (pH 7.4) containing 10% DMSO

It can be seen that Im7, which is according to this invention, provides a higher kinetic constant value, both with BCN and with TMTH, than similar iminosydnones which bear either no F group (see Im5) or a different F group (see Im6). These results further show that the iminosydnones comprising a carbamate function at F' position (compare Im11 with Im3) and the iminosydnones comprising a halogen atom in position 4 (compare Im9 with Im10 and Im11 with Im12) afford the fastest reactions. In particular, 4-bromo-6-carbamate iminosydnones were found to react with the cyclooctyne BCN with a constant of 0.15 $M^{-1}.sec^{-1}$ which allows the reaction to proceed at concentration as low as 1 μM.

Second order reaction rate was determined by plotting ln([A]/[B])/([A] - [B]) versus time and analyzing by linear regression **(Equation).** Second order rate constant corresponds to the determined slope.

$$\frac{\ln\left(\frac{[A]}{[B]}\right)}{[A]-[B]} = kt + const$$

**Equation.** [A] - concentration of iminosydnones (M); [B] - concentration of BCN (M); t - reaction time (sec); k - reaction rate ($M^{-1} \cdot sec^{-1}$)

Linear regression curves for sydnones Im2, Im6 and Im7 are illustrated in figures 2 and 3.

**Claims**

1. Process for the preparation of a conjugate of formula (V):

(V) 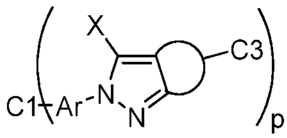 ,

wherein:

C1 is a compound of interest selected from an antibody, a protein, a drug, a fluorophore, a nanoparticle and a polymer,
C3 is a compound of interest selected from a drug, a fluorophore, a nanoparticle and a polymer,
Ar is an optionally substituted phenyl group,
X is hydrogen,
p is an integer from 1 to 100,
wherein C1 and Ar are covalently linked by a functional group,
and for releasing a derivative of a compound of interest C2 of formula (VII):

(VII) 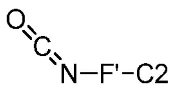 ,

wherein C2 is a compound of interest selected from the group consisting of a fluorophore, a drug, an antibody, a protein, a nanoparticle and a polymer and F' is CO, comprising a step of contacting a conjugate of formula (IV)

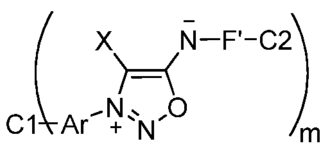

(IV)

wherein C1, C2, Ar, X and F' are as defined above in this claim, C2 and F' are covalently linked by a functional group and m is an integer from 1 to 100 with a compound comprising a strained alkyne moiety of formula (VI) :

(VI) 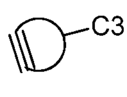

wherein C3 is as defined above in this claim.

2. Process of preparation according to claim 1, wherein the process comprises a preliminary step of covalent bonding of the strained alkyne moiety to the compound of interest C3.

**3.** Process according to any of claims 1 and 2, wherein C1 is a nanoparticle or an antibody, C2 is a drug and C3 is a fluorophore.

**4.** Conjugate of formula (IV): wherein

(IV) 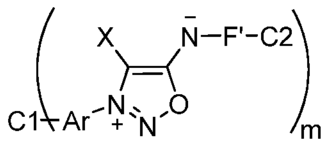 ,

C1, Ar, X, F', C2 and m are as defined in Claim 1.

## Patentansprüche

**1.** Verfahren zur Herstellung eines Konjugats der Formel (V):

(V) 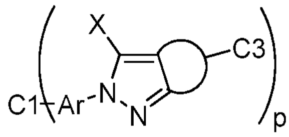 ,

wobei:

C1 eine Verbindung von Interesse ist, ausgewählt aus einem Antikörper, einem Protein, einem Arzneimittel, einem Fluorophor, einem Nanopartikel und einem Polymer,
C3 eine Verbindung von Interesse ist, ausgewählt aus einem Arzneimittel, einem Fluorophor, einem Nanopartikel und einem Polymer,
Ar eine gegebenenfalls substituierte Phenylgruppe ist,
X Wasserstoff ist,
p eine ganze Zahl von 1 bis 100 ist,
wobei C1 und Ar kovalent durch eine funktionelle Gruppe gekoppelt sind,
und zum Freisetzen eines Derivats einer Verbindung von Interesse C2 der Formel (VII):

(VII) 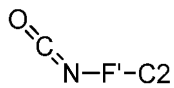 ,

wobei C2 eine Verbindung von Interesse ist, ausgewählt aus der Gruppe bestehend aus einem Fluorophor, einem Arzneimittel, einem Antikörper, einem Protein, einem Nanopartikel und einem Polymer, und F' CO ist, umfassend einen Schritt des Kontaktierens eines Konjugats der Formel (IV)

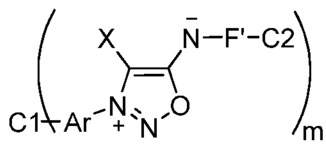

(IV)

wobei C1, C2, Ar, X und F' wie vorstehend in diesem Anspruch definiert sind, C2 und F' kovalent durch eine funktionelle Gruppe gekoppelt sind und m eine ganze Zahl von 1 bis 100 ist,
mit einer Verbindung, die einen gestreckten Alkinrest der Formel (VI) umfasst:

(VI) 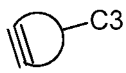

wobei C3 wie oben in diesem Anspruch definiert ist.

**2.** Verfahren zur Herstellung nach Anspruch 1, wobei das Verfahren einen vorherigen Schritt der kovalenten Bindung des gestreckten Alkinrestes an die Verbindung von Interesse C3 umfasst.

**3.** Verfahren nach einem der Ansprüche 1 und 2, wobei C1 ein Nanopartikel oder ein Antikörper ist, C2 ein Arzneimittel ist und C3 ein Fluorophor ist.

**4.** Konjugat nach Formel (IV):

(IV) 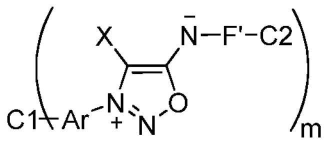 ,

wobei
C1, Ar, X, F', C2 und m wie in Anspruch 1 definiert sind.

## Revendications

**1.** Procédé de préparation d'un conjugué de formule (V) :

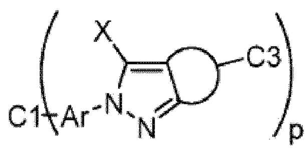 (V),

dans laquelle :

C1 est un composé d'intérêt choisi parmi un anticorps, une protéine, un médicament, un fluorophore, une nanoparticule et un polymère,
C3 est un composé d'intérêt choisi parmi un médicament, un fluorophore, une nanoparticule et un polymère,
Ar est un groupe phényle éventuellement substitué,
X est un hydrogène,
p est un entier allant de 1 à 100,
où C1 et Ar sont liés de manière covalente par un groupe fonctionnel,
et de libération d'un dérivé d'un composé d'intérêt C2 de formule (VII) :

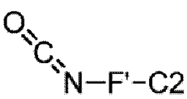 (VII),

dans laquelle C2 est un composé d'intérêt choisi dans le groupe constitué d'un fluorophore, un médicament, un anticorps, une protéine, une nanoparticule et un polymère, et F' est CO, comprenant une étape de mise en contact d'un conjugué de formule (IV) :

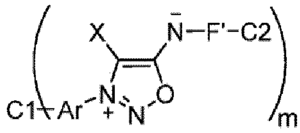 (IV),

dans laquelle C1, C2, Ar, X et F' sont tels que définis ci-dessus dans cette revendication, C2 et F' sont liés de manière covalente par un groupe fonctionnel et m est un entier allant de 1 à 100,
avec un composé comprenant un fragment alcyne tendu de formule (VI) :

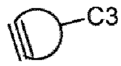 (VI),

dans laquelle C3 est tel que défini ci-dessus dans cette revendication.

2. Procédé de préparation selon la revendication 1, dans lequel le procédé comprend une étape préliminaire de liaison covalente du fragment alcyne tendu au composé d'intérêt C3.

3. Procédé selon la revendication 1 ou 2, dans lequel C1 est une nanoparticule ou un anticorps, C2 est un médicament, et C3 est un fluorophore.

4. Conjugué de formule (IV) :

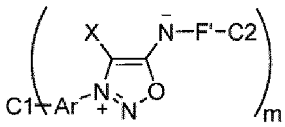 (IV),

dans laquelle C1, Ar, X, F', C2 et m sont tels que définis dans la revendication 1.

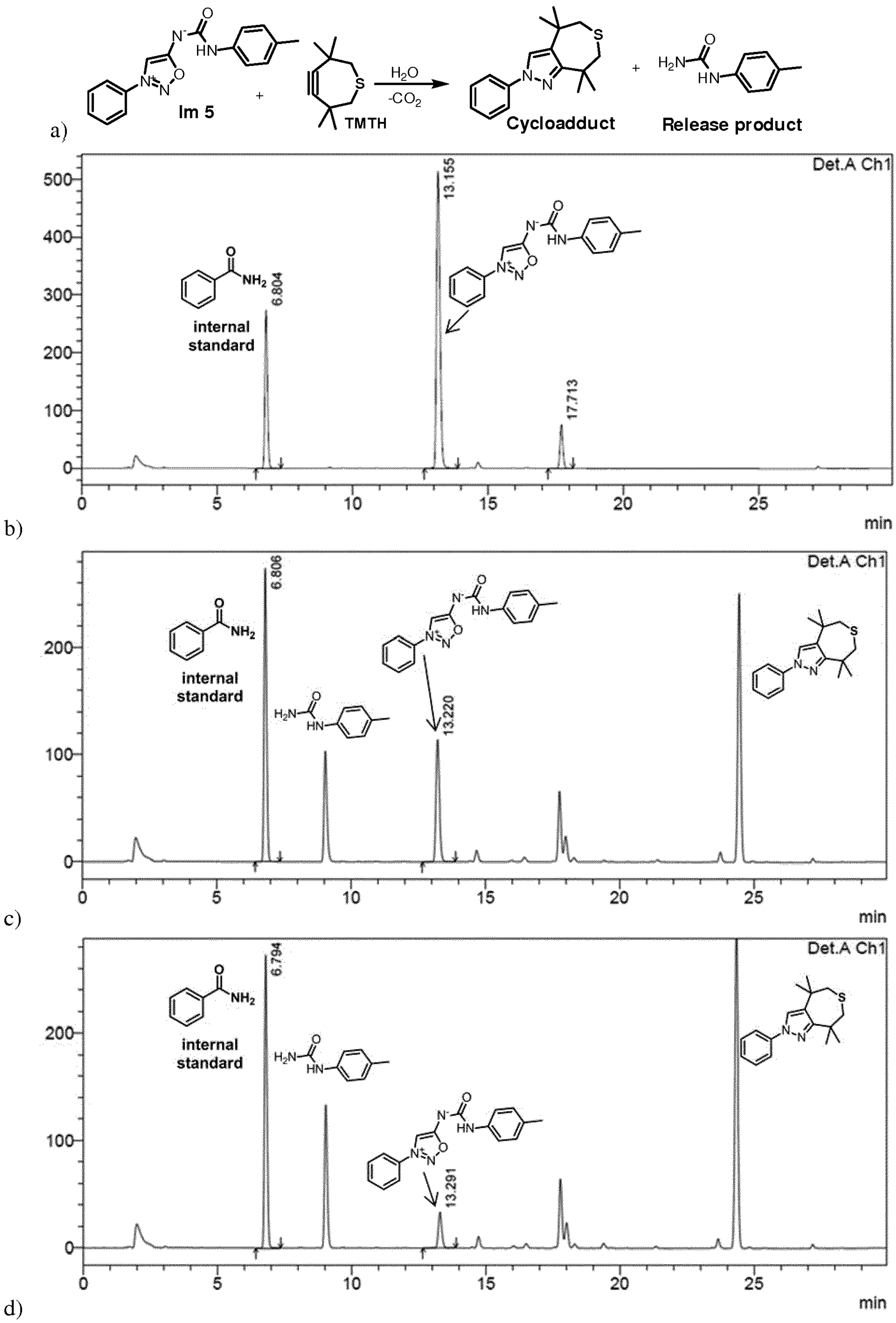
a)
Im 5
TMTH
$H_2O$
$-CO_2$
Cycloadduct
Release product
b)
Det.A Ch1
internal standard
6.804
13.155
17.713
min
c)
Det.A Ch1
internal standard
6.806
13.220
min
d)
Det.A Ch1
internal standard
6.794
13.291
min

Figure 1

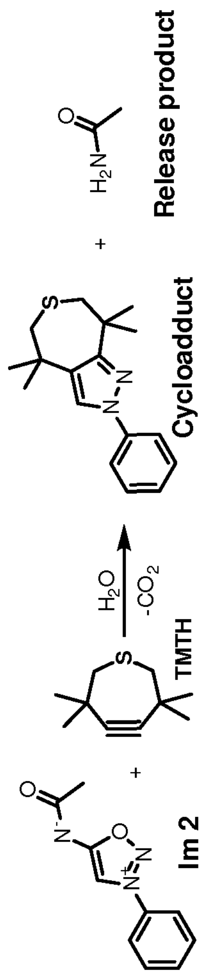


Figure 2a

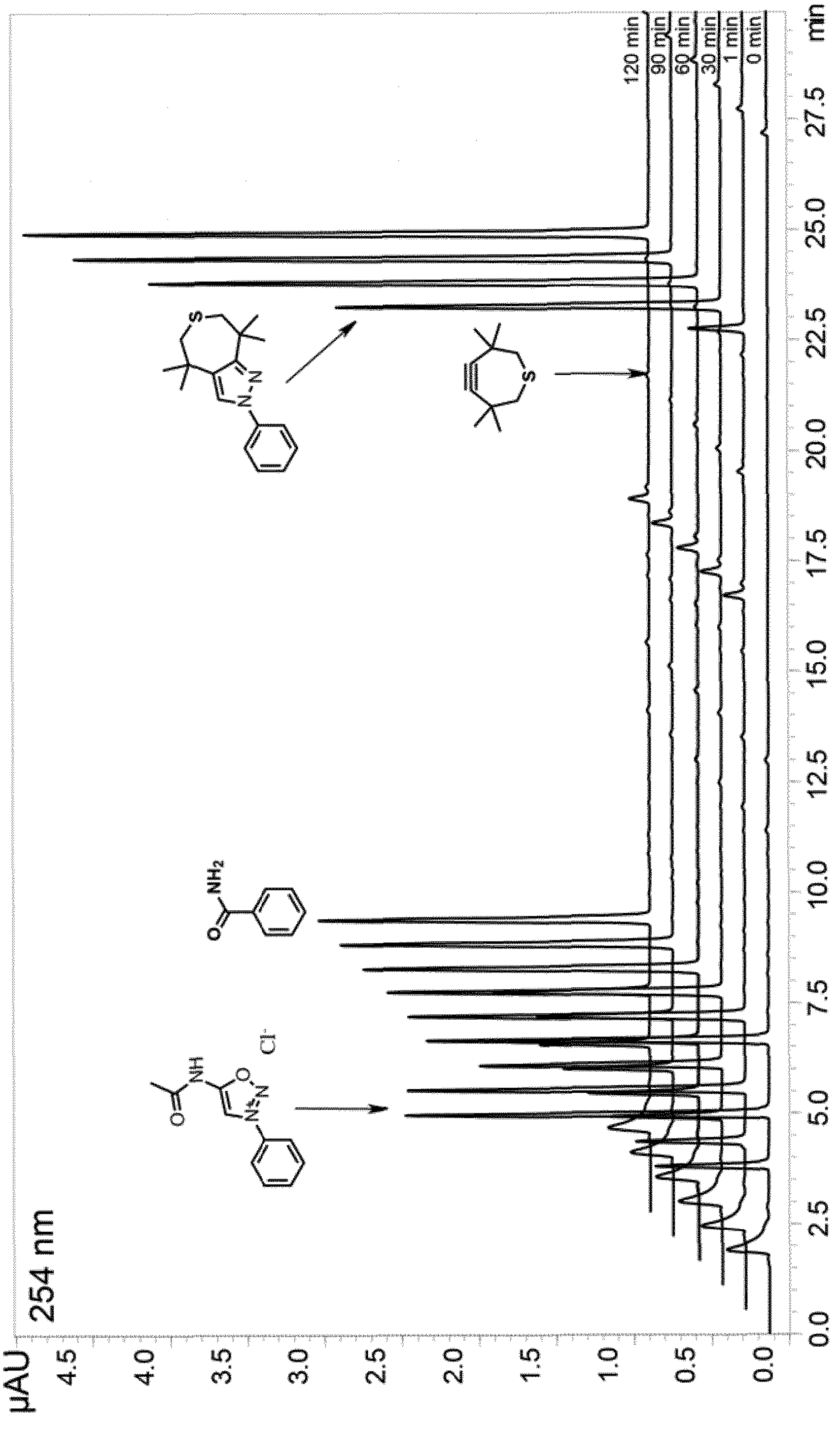


Figure 2b

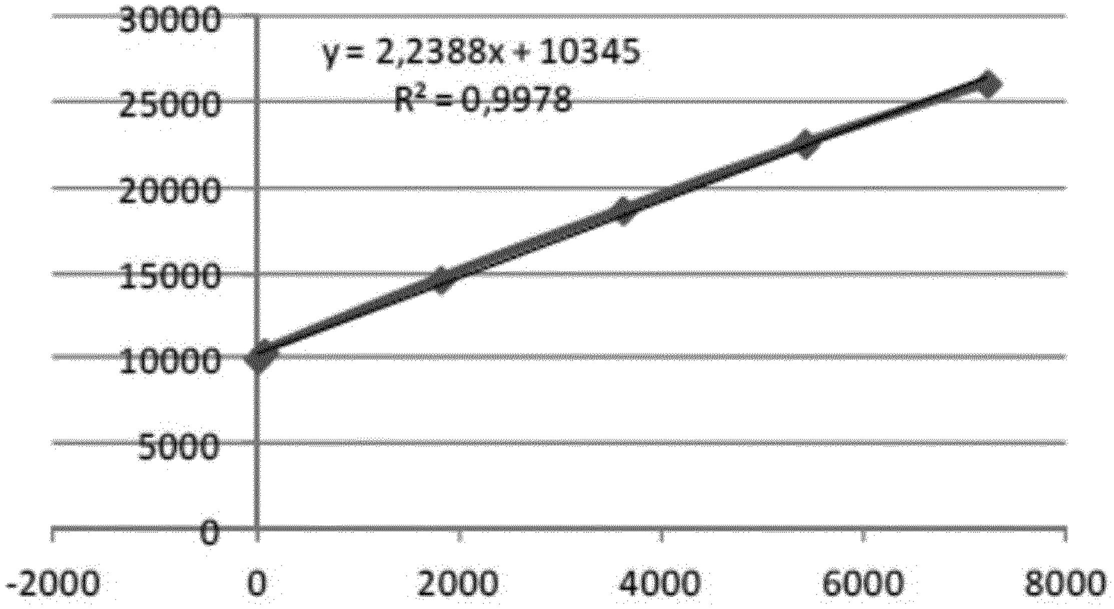
y = 2,2388x + 10345
$R^2$ = 0,9978
30000
25000
20000
15000
10000
5000
0
-2000
0
2000
4000
6000
8000

Figure 2c

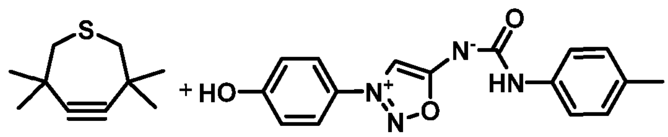

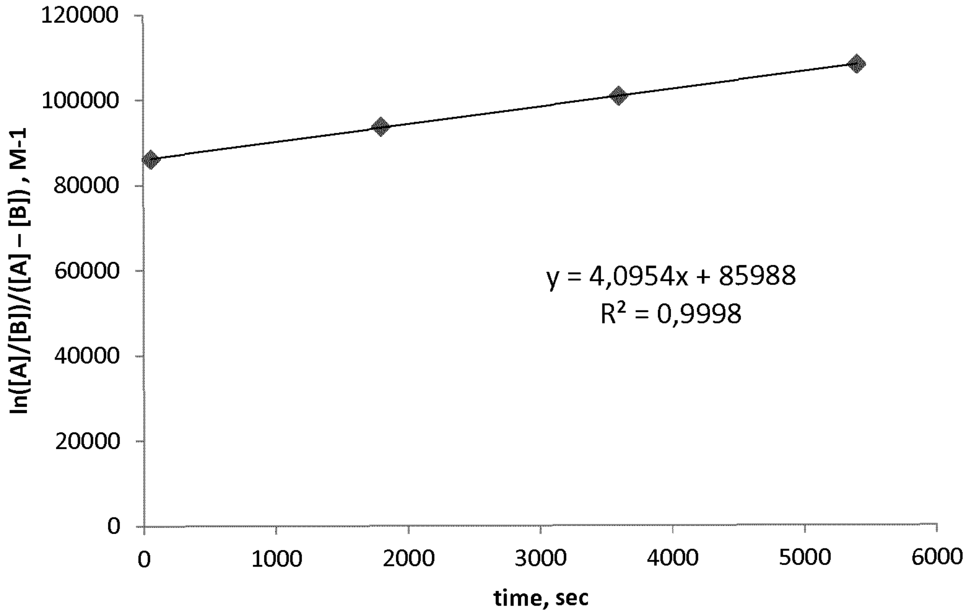
120000
100000
80000
60000
40000
20000
0
0
1000
2000
3000
4000
5000
6000
time, sec
ln([A]/[B])/([A] – [B]) , M-1
y = 4,0954x + 85988
R² = 0,9998

a)

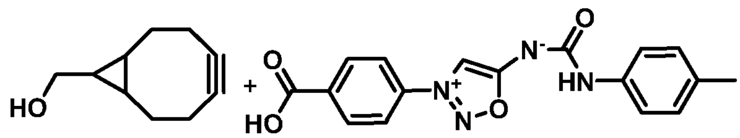

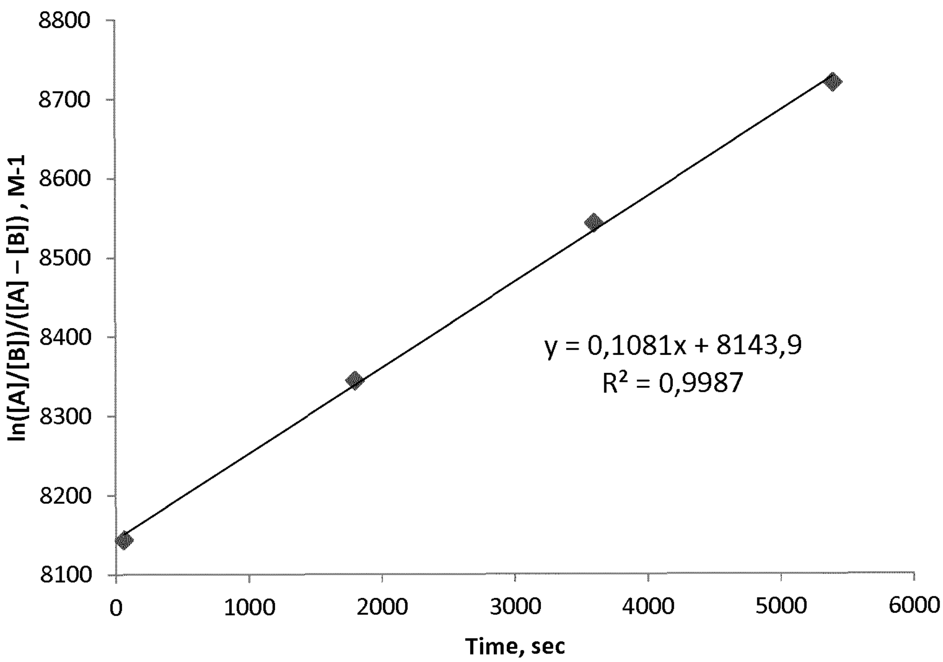
8800
8700
8600
8500
8400
8300
8200
8100
0
1000
2000
3000
4000
5000
6000
Time, sec
ln([A]/[B])/([A] – [B]) , M-1
y = 0,1081x + 8143,9
R² = 0,9987

b)

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Angew. Chem. Int. Ed.,* 2013, vol. 52, 14112-14116
- **KOLODYCH et al.** *Angew. Chem. Int. Ed.,* 2013, vol. 52, 12056-12060
- **WALLACE.** *Chin Chem. Sci.,* 2014, vol. 5, 1742-1744
- *J. Pharm. Sci.,* 1977, vol. 66, 2
- Handbook of Pharmaceutical Salts: Properties, Selection, and Use. 2002